# EUROPÄISCHE PATENTSCHRIFT

(11) **EP 0 820 986 B1**
(45) Veröffentlichungstag und Bekanntmachung des Hinweises auf die Patenterteilung: **02.10.2002**
(21) Anmeldenummer: 97112547.1
(22) Anmeldetag: 22.07.1997
(51) Int. Cl.: C07D 213/79, C07D 213/80, C07D 213/61

(54) **Verfahren zur Herstellung von Pyridincarbonsäureestern**
Process for the preparation of pyridine carboxylic acid esters
Procédé pour la préparation d'esters d'acides pyridine carboxyliques

(30) Priorität: 23.07.1996 CH 184196
(43) Veröffentlichungstag der Anmeldung: 28.01.1998
(73) Patentinhaber: Lonza AG, 3930 Visp (CH)
(72) Erfinder: Bessard, Yves, Dr., 3960 Sierre (Kanton Wallis) (CH); Stucky, Gerhard, Dr., 3902 Brig-Glis (Kanton Wallis) (CH); Roduit, Jean-Paul, Dr., 3979 Grône (Kanton Wallis) (CH)
(74) Vertreter: Winter, Brandl, Fürniss, Hübner, Röss, Kaiser, Polte Partnerschaft

(56) Entgegenhaltungen:
- EP-A- 0 282 266
- EP-A- 0 353 187

## Beschreibung

Die vorliegende Erfindung betrifft ein Verfahren zur Herstellung von Pyridincarbonsäureestern durch Umsetzung von halogenierten Pyridinen mit Kohlenmonoxid und einem C₁-C₄-Alkanol in Gegenwart einer schwachen Base und eines Katalysators.

Pyridincarbonsäureester sind wichtige Zwischenprodukte, z B fur die Herstellung von Herbiziden (EP-A-0 488 474) und für die Herstellung von Arzneimitteln gegen fibrotische Erkrankungen (EP-A-0 673 932)

Die erfindungsgemäss herstellbaren Pyridincarbonsäureester besitzen die allgemeine Formel 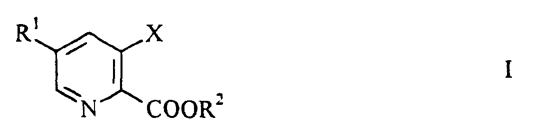 worin R¹ Wasserstoff, eine C₁-C₆-Alkylgruppe, eine C₁-C₄-Alkoxycarbonylgruppe oder eine C₁-C₄-Alkoxymethylgruppe und R² eine C₁-C₄-Alkylgruppe bedeutet und X Chlor oder Brom ist.

Verfahren zur Herstellung von Pyridincarbonsäurestern durch Carbonylierungs-Reaktionen, ausgehend von Mono- und Dihalopyridinen, sind in der Literatur bekannt (EP-A-0 353 187, EP-A-0 282 266, WO 93/18005; US-A-4 128 554; Shokubai (Catalysis Society of Japan) 36 (1994), 380). Nachteilig bei diesen Verfahren ist, dass die Pyridincarbonsäureester nur in massigen Ausbeuten erhalten werden Nachteilig bei den in EP-A-0 282 266 und WO 93/18005 beschriebenen Verfahren ist weiterhin, dass die als Ausgangsmaterial eingesetzten Dihalopyridine nur wenig selektiv carbonyliert werden. In einem weiteren Carbonylierungsverfahren (J. Mol. Cat., 66 (1991), 277) werden, ausgehend von Monohalopyridinen, hohe Ausbeuten an Pyridincarbonsäureestern erzielt, für die Umsetzung jedoch lange Reaktionszeiten benötigt.

Die Aufgabe der vorliegenden Erfindung war daher, ein okonomisches Verfahren zur Verfugung zu stellen, mit welchem, ausgehend von Dihalopyridinen, selektiv monocarbonylierte Pyridincarbonsäureester der allgemeinen Formel I in hohen Ausbeuten herzustellen sind

Erfindungsgemäss wird diese Aufgabe durch das Verfahren nach Anspruch 1 gelöst. Darin werden 2,3-Dihalopyridine der allgemeinen Formel 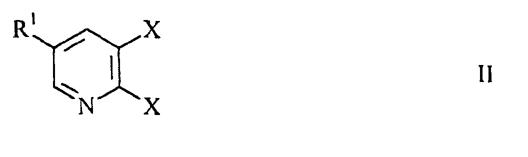 worin R¹ und X die oben genannte Bedeutung haben, mit Kohlenmonoxid und einem C₁-C₄-Alkanol in Gegenwart einer schwachen Base, ausgewählt aus der Gruppe bestehend aus Alkali- oder Erdalkaliacetaten, -hydrogencarbonaten und -hydrogenphosphaten, und eines Komplexes von Palladium mit einem Bis-diphenylphosphin der allgemeinen Formel 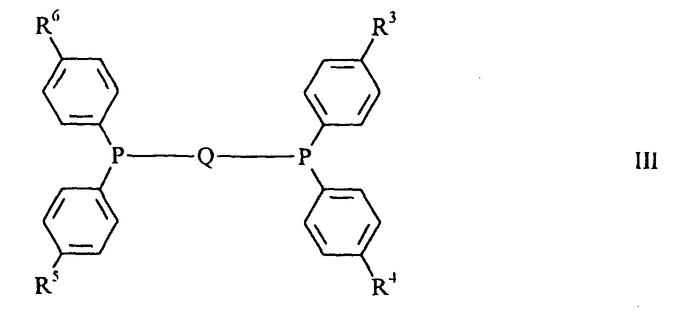 worin Q eine C₃-C₆-Alkandiylgruppe oder eine 1,1'-Ferrocendiylgruppe mit gegebenenfalls C₁-C₄-Alkyl- oder Aryl- substituierten Cyclopentadienylgruppen und R³ bis R⁶ unabhängig voneinander Wasserstoff, C₁-C₄-Alkyl, C₁-C₄-Alkoxy, Fluormethyl, Fluor, Aryl, Phenoxy, Nitril oder Dialkylamino bedeuten, zur Reaktion gebracht.

R¹ hat die Bedeutung von Wasserstoff oder einer geradkettigen oder verzweigten Alkylgruppe mit 1 bis 6 C-Atomen, einer Alkoxycarbonylgruppe mit einer geradkettigen oder verzweigten Alkylgruppe mit 1 bis 4 C Atomen oder einer Alkoxymethylgruppe mit einer geradkettigen oder verzweigten Alkylgruppe mit 1 bis 4 C Atomen Namentlich erwähnt seien Methyl und Ethyl, *n*-Propyl, *i*-Propyl, *n-*, *i-, t*-Butyl, Pentyl und seine Isomeren sowie Hexyl und seine Isomeren, Methoxycarbonyl, Ethoxycarbonyl, *n-, i*-Propoxycarbonyl, *n-, i-, t*-Butoxycarbonyl, Methoxymethyl, Ethoxymethyl, *n-, i*-Propoxymethyl, *n*-, *i*-, *t*-Butoxymethyl Besonders bevorzugt hat R¹ die Bedeutung von Methoxycarbonyl und Methoxymethyl R² ist eine geradkettige oder verzweigte Alkylgruppe mit 1 bis 4 C-Atomen Namentlich erwähnt seien Methyl, Ethyl, *n*-Propyl, *i*-Propyl, *n*-, *i*-, *t*-Butyl Besonders bevorzugt hat R² die Bedeutung von Methyl und Ethyl, X hat die Bedeutung von Chlor oder Brom, besonders bevorzugt hat X die Bedeutung von Chlor.

Ein 2,3-Dihalopyridin der allgemeinen Formel II kann auf einfache Weise, ausgehend von 2-Chlorpyridin-1-oxid (US-A-5 334 724) oder ausgehend von 6-Hydroxynikotinsaure (CH-PS-664 754; Encyclopedia of Reagents for Organic Synthesis, Vol 4, ed. L.A Paquette, John Wiley & Sons, Chichester (1995), 2769-71) hergestellt werden

Als Alkanol wird ein geradkettiger oder verzweigter aliphatischer Alkohol mit 1 bis 4 C-Atomen eingesetzt. Namentlich erwähnt seien Methanol, Ethanol, *n*-, *i*-Propanol, *n*-, *i*-, *t*-Butanol. Besonders bevorzugt sind Methanol und Ethanol

Als Alkali- und Erdalkaliacetate, -hydrogencarbonate oder -hydrogenphosphate können beispielsweise Natriumacetat, Kaliumacetat, Magnesiumacetat, Calciumacetat, Natriumhydrogencarbonat, Kaliumhydrogencarbonat, Magnesiumhydrogencarbonat. Calciumhydrogencarbonat, Dinatriumhydrogenphosphat, Dikaliumhydrogenphosphat, Magnesiumhydrogenphosphat und Calciumhydrogenphosphat eingesetzt werden. Besonders geeignet ist Natriumacetat.

Der katalytisch wirksame Palladium-Bis-diphenylphosphin-Komplex wird vorteilhaft *in situ* gebildet, indem ein Pd(II) Salz (z. B. das Chlorid oder das Acetat, bevorzugt das Acetat), oder ein geeigneter Pd(II)-Komplex (z B. Bis(triphenylphosphin)palladium(II)chlorid) mit dem Diphosphin zur Reaktion gebracht wird Das Palladium wird vorzugsweise in einer Menge von 0,05 bis 0,4 Mol-% Pd(II), bezogen auf die Halogenverbindung (II), eingesetzt. Das Diphosphin wird vorteilhaft im Uberschuss (bezogen auf Pd) eingesetzt, vorzugsweise in einer Menge von 0,2 bis 5 Mol-%, ebenfalls bezogen auf die Halogenverbindung (II)

Als Bis-diphenylphosphine (III) werden vorteilhaft solche eingesetzt, in denen Q eine geradkettige oder verzweigte Alkandiylgruppe mit 3 bis 6 C-Atomen bedeutet. Namentlich erwähnt seien Propan-1,3-diyl, Propan-1,2-diyl, Butan-1,4-diyl, Butan-1,3-diyl, Butan-1,2-diyl, Pentandiyl und seine Isomeren sowie Hexandiyl und seine Isomeren. Vorzugsweise werden solche eingesetzt, in denen Q eine geradkettige Alkandiylgruppe mit 3-6 C-Atomen bedeutet Namentlich erwähnt seien Propan-1,3-diyl, Butan-1,4-diyl, Pentan-1,5-diyl sowie Hexan-1,6-diyl. Besonders bevorzugt ist 1,4-Bis(diphenyl-phosphino)butan Ebenfalls vorteilhaft eingesetzt werden Bis-diphenylphosphine (III), in denen Q eine 1,1'-Ferrocendiylgruppe mit gegebenenfalls C₁-C₄-Alkyl- oder Aryl- substituierten Cyclopentadienylgruppen bedeutet. Als C₁-C₄-Alkyl-Substituenten werden bevorzugt Methyl, Ethyl, *n*-Propyl, *i*-Propyl, *n*-, *i*-, *t*-Butyl eingesetzt, besonders bevorzugt sind Methyl und Ethyl. Als Aryl-Substituenten werden bevorzugt Phenyl und gegebenenfalls substituiertes Phenyl eingesetzt. Unter substituiertem Phenyl sind insbesondere Gruppen wie (*p*)-Fluorphenyl, (*p*)-Methoxyphenyl, (*p*)-Tolyl oder (*p*)-Trifluormethylphenyl zu verstehen R³ bis R⁶ der eingesetzten Bis-diphenylphosphine (III) bedeuten unabhängig voneinander Wasserstoff, C₁-C₄-Alkyl, C₁-C₄-Alkoxy, Fluormethyl, Fluor, Aryl, Phenoxy, Nitril oder Dialkylamino.

Als C₁-C₄-Alkyl-Substituenten werden vorteilhaft Methyl, Ethyl, *n*-Propyl, *t*-Propyl, *n*-, *i-, t*-Butyl eingesetzt, besonders bevorzugt sind Methyl und Ethyl Als C₁-C₄-Alkoxy-Substituenten werden vorteilhaft Methoxy, Ethoxy, *n*-,*i*-Propoxy, *n*-, *i*-, *t*-Butoxy eingesetzt, besonders bevorzugt sind Methoxy und Ethoxy. Als Aryl-Substituenten werden vorteilhaft Phenyl und gegebenenfalls substituiertes Phenyl eingesetzt. Unter substituiertem Phenyl sind die oben genannten Gruppen zu verstehen. Als Phenoxy-Substituenten werden vorteilhaft Phenoxy und gegebenenfalls substituiertes Phenoxy eingesetzt. Unter substituiertem Phenoxy sind insbesondere Gruppen wie (*p*)-Fluorphenoxy, (*p*)-Methoxyphenoxy, Benzyloxy oder (*p*)-Trifluormethylphenoxy zu verstehen.

Als Dialkylamino-Substituenten werden bevorzugt Amine mit C₁-C₂-Alkyl-Resten eingesetzt Namentlich erwähnt seien Dimethylamino und Diethylamino

Die Reaktion wird vorteilhaft in einem Lösungsmittel durchgefuhrt Als Lösungsmittel können sowohl unpolare, wie beispielsweise Toluol oder Xylol, als auch polare organische Lösungsmittel wie beispielsweise Acetonitril, Tetrahydrofuran oder *N,N*-Dimethylacetamid eingesetzt werden.

Die Reaktion wird vorteilhaft bei einer Reaktionstemperatur von 100 bis 250°C, vorzugsweise bei 140 bis 195°C und einem Kohlenmonoxiddruck von vorteilhaft 1 bis 200 bar, vorzugsweise 5 bis 50 bar durchgeführt. Nach einer Umsetzungszeit von ublicherweise 1 bis 6 Stunden erhält man die Verbindung der allgemeinen Formel I in hohen Ausbeuten

Mit dem erfindungsgemassen Verfahren können 3-Halo-5-(methoxymethyl)-2-pyridincarbonsaureester hergestellt werden, wie z. B ausgehend von 2,3-Dichlor-5-(methoxymethyl)pyridin, 3-Chlor-5-(methoxymethyl)-2-pyridincarbonsäuremethylester.

Die folgenden Beispiele verdeutlichen die Durchführung des erfindungsgemassen Verfahrens

### Beispiele:

### Beispiel 1a)

### Herstellung von 2,3-Dichlor-5-(methoxymethyl)pyridin

Unter Argon wurden 4,14 g (23 mmol) Natriummethanolat (30%ige Lösung in Methanol) bei Raumtemperatur innerhalb von 5 min zu einer Lösung von 4,11 g (20,9 mmol) 2,3-Dichlor-5-(chlormethyl)pyridin (hergestellt aus 2,3-Dichlor-5-(hydroxymethyl)pyridin durch Umsetzung mit Thionylchlorid) in 40 ml Methanol getropft. Anschliessend wurde das Reaktionsgemisch 3 h auf 60 °C erwärmt. Nach dem Ende der Reaktion wurde das Lösungsmittel abdestilliert, der Rückstand mit 100 ml Wasser versetzt und mit Dichlormethan (3 × 75 ml) extrahiert. Die organische Phase wurde über Magnesiumsulfat getrocknet und eingedampft.
Ausbeute: 4,06 g (90,4%) gelbes Öl, Gehalt (GC) 90,8%

Zur Analyse wurde das Produkt mit Hexan/Ethylacetat (3:1) an Kieselgel chromatographiert.
- 1H NMR (CDCl₃) δ =: 8,25 (s, 1H);
7,78 (s, 1H);
4,46 (s, 2H);
3,43 (s, 3H).
- MS (*m*/*z*):: 192 (M⁺); 176; 161; 148; 124; 112.

### Beispiel 1b)

### Herstellung von 3-Chlor-5-(methoxymethyl)-2-pyridincarbonsäuremethylester

In einem Autoklaven wurden 1,16 g (5,6 mmol) 2,3-Dichlor-5-(methoxymethyl)pyridin, 72 mg 1,4-Bis(diphenylphosphino)butan (3 Mol% bezogen auf 2,3-Dichlor-5-(methoxymethyl)-pyridin), 8 mg Bis(triphenylphosphin)palladium(II)chlorid (0,2 Mol% bezogen auf 2,3-Dichlor-5-(methoxymethyl)pyridin), 1,39 g (17 mmol) Natriumacetat und 16 ml Methanol vorgelegt. Der Autoklav wird mehrmals mit Kohlenmonoxid durchströmt um die Luft gegen Kohlenmonoxid auszutauschen.

Anschliessend wird Kohlenmonoxid mit 15 bar in den Autoklaven gepresst. Das Reaktionsgemisch wird auf 165 °C (Badtemperatur) erhitzt und 6 Stunden gerührt. Nach dem Abkühlen auf Raumtemperatur wird das Rohprodukt unter Vakuum (30 mbar) aufkonzentriert, an Kieselgel 60 (Laufmittel Hexan/Ethylacetat im Verhältnis 3:1) chromatographiert, mit Gaschromatographie analysiert und mit ¹H-NMR und MS/GC-MS charakterisiert.
Ausbeute: 1,05 g (87%) Farbloses Öl
MS; *m*/*z*: 215 (M⁺); 185; 157
- ¹H-NMR (CDCl₃): δ =: 8,50 (s, 1H);
7,82 (s, 1H);
4,53 (s, 2H);
4,01 (s, 3H);
3,45 (s, 3H).

### Beispiel 2

### Herstellung von 3-Chlor-5-(methoxymethyl)-2-pyridincarbonsäuremethylester

Es wurde verfahren wie in Beispiel 1b) beschrieben, jedoch wurde anstelle von 16 ml Methanol, 16 ml Tetrahydrofuran und 12 mmol Methanol eingesetzt. Nach einer Reaktionszeit von 6 Stunden bei einer Badtemperatur von 195 °C wurden 0,67 g (55%) farbloses Öl erhalten.

### Beispiel 3

### Herstellung von 3-Chlor-5-(methoxymethyl)-2-pyridincarbonsäureethylester

Es wurde verfahren wie in Beispiel 1b) beschrieben, jedoch wurde anstelle von Methanol das gleiche Volumen Ethanol und anstelle von 1,4-Bis(diphenylphosphino)butan die gleiche molare Menge 1,1'-Bis(diphenylphosphino)ferrocen eingesetzt. Nach einer Reaktionszeit von 2 Stunden bei einer Badtemperatur von 162 °C wurden 0,54 g (47%) farbloser Feststoff erhalten.
MS; *m*/*z*: 229 (M⁺); 186; 157
- ¹H-NMR (CDCl₃): δ =: 8,50 (s, 1H);
7,80 (s, 1H);
4,51 (s, 2H);
4,49 (q, 2H);
3,43 (s, 3H);
1,42 (t, 3H).

### Beispiel 4

### Herstellung von 3-Chlor-2,5-pyridindicarbonsäuredimethylester

Es wurde verfahren wie in Beispiel 2 beschrieben, jedoch wurde anstelle von 2,3-Dichlor-5-(methoxymethyl)pyridin die gleiche molare Menge 2,3-Dichlor-5-pyridincarbonsäuremethylester eingesetzt. Nach einer Reaktionszeit von 6 Stunden bei einer Badtemperatur von 160 °C wurden 1,20 g (73%) gelbes Produkt erhalten.
Schmp.: 35,5-36°C
MS; *m*/*z*: 229 (M⁺); 185; 157
- ¹H-NMR (CDCl₃): δ =: 9,11 (s, 1H);
8,40 (s, 1H);
4,05 (s, 3H);
3,99 (s, 3H).

### Beispiel 5

### Herstellung von 3-Chlor-2-pyridincarbonsäureethylester

Es wurde verfahren wie in Beispiel 1b) beschrieben, jedoch wurde anstelle von 1,16 g (5,6 mmol) 2,3-Dichlor-5-(methoxymethyl)pyridin 0,76 g (5,0 mmol) 2,3-Dichlorpyridin, anstelle von 72 mg (0,178 mmol) 1,4-Bis(diphenylphosphino)butan 83,2 mg (0,15 mmol) 1,1'-Bis(diphenylphosphino)ferrocen, anstelle von 8 mg (0,0112 mmol) Bis(triphenylphosphin)-palladium(II)chlorid 2,2 mg (0,01 mmol) Pd(II)-Acetat, anstelle von 16 ml Methanol 14,5 ml Ethanol und anstelle von 17 mmol 10,5 mmol Natriumacetat eingesetzt. Nach einer Reaktionszeit von 1 Stunde bei einer Badtemperatur von 140 °C wurden 0,83 g (88%) ölige Substanz erhalten.
MS; *m*/*z*: 185 (M⁺); 140; 113; 85; 76
- ¹H-NMR (CDCl₃): δ =: 8,58 (d, 1H);
7,81 (d, 1H);
7,38 (dd, 1H);
4,54 (q, 2H);
1,44 (t, 3H).

## Patentansprüche

1. Verfahren zur Herstellung von Pyridincarbonsäureestern der allgemeinen Formel 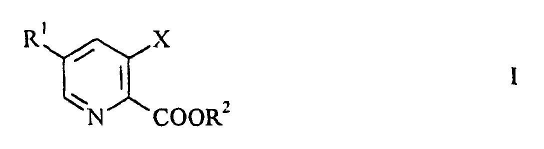 worin R¹ Wasserstoff, eine C₁-C₆-Alkylgruppe, eine C₁-C₄-Alkoxycarbonylgruppe oder eine C₁-C₄-Alkoxymethylgruppe und R² eine C₁-C₄-Alkylgruppe bedeutet und X Chlor oder Brom ist, **dadurch gekennzeichnet, dass** ein 2,3-Dihalopyridin der allgemeinen Formel 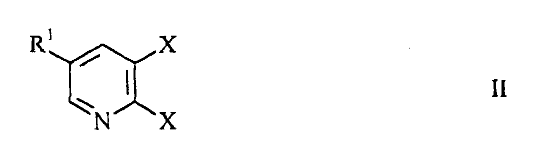 worin R¹ und X die oben genannte Bedeutung haben, mit Kohlenmonoxid und einem C₁-C₄-Alkanol in Gegenwart einer schwachen Base, ausgewahlt aus der Gruppe bestehend aus Alkali- und Erdalkaliacetaten, -hydrogencarbonaten und -hydrogenphosphaten, und eines Komplexes von Palladium mit einem Bis-diphenylphosphin der allgemeinen Formel 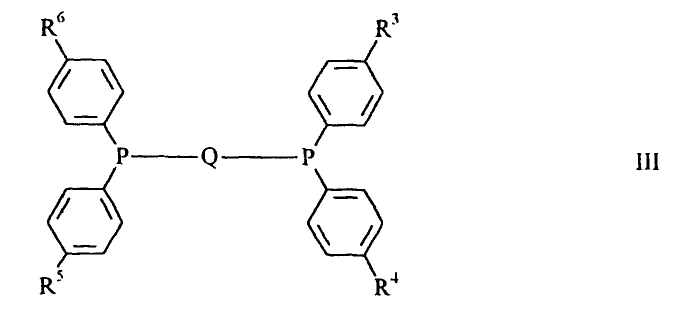 worin Q eine C₃-C₆-Alkandiylgruppe oder eine 1,1'-Ferrocendiylgruppe mit gegebenenfalls C₁-C₄-Alkyl- oder Aryl-substituierten Cyclopentadienylgruppen und R³ bis R⁶ unabhängig voneinander Wasserstoff, C₁-C₄-Alkyl, C₁-C₄-Alkoxy, Fluormethyl, Fluor, Aryl, Phenoxy, Nitril oder Dialkylamino bedeuten, zur Reaktion gebracht wird

2. Verfahren nach Anspruch 1, **dadurch gekennzeichnet, dass** R¹ eine Methoxycarbonylgruppe oder Methoxymethylgruppe ist.

3. Verfahren nach Anspruch 1 oder 2, **dadurch gekennzeichnet, dass** die Reaktion in einem unpolaren oder polaren organischen Lösungsmittel durchgeführt wird.

4. Verfahren nach irgendeinem der Ansprüche 1 bis 3, **dadurch gekennzeichnet, dass** Palladium in Form von Bis(triphenylphosphin)palladium(II)chlorid oder Palladium(II)acetat eingesetzt wird.

5. Verfahren nach irgendeinem der Ansprüche 1 bis 4, **dadurch gekennzeichnet, dass** der Kohlenmonoxiddruck 1 bis 200 bar ist.

6. Verfahren nach irgendeinem der Ansprüche 1 bis 5, **dadurch gekennzeichnet, dass** die Reaktionstemperatur 100 bis 250 °C ist.

## Claims

1. Process for the preparation of pyridinecarboxylic acid esters corresponding to the general formula 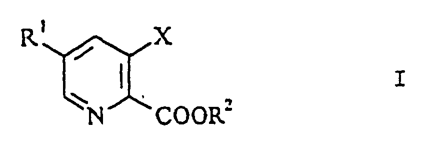 wherein R¹ denotes hydrogen, a C₁-C₆-alkyl group, a C₁-C₄-alkoxycarbonyl group or a C₁-C₄-alkoxymethyl group and R² denotes a C₁-C₄-alkyl group and X is chlorine or bromine, **characterised in that** a 2,3-dihalopyridine corresponding to the general formula 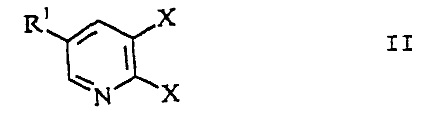 wherein R¹ and X have the meanings given above, is reacted with carbon monoxide and with a C₁-C₄-alkanol in the presence of a weak base selected from alkali metal acetates and alkaline-earth acetates, alkali metal hydrogen carbonates and alkaline-earth hydrogen carbonates and alkali metal hydrogen phosphates and alkaline-earth hydrogen phosphates, and of a complex of palladium with a bisphenylphosphine corresponding to the general formula 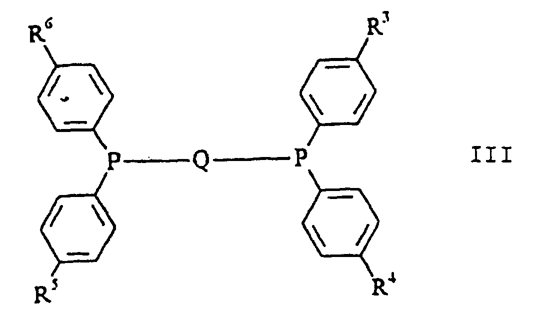 wherein Q denotes a C₃-C₆-alkanediyl group or a 1,1'-ferrocenediyl group having optionally C₁-C₄-alkyl- or aryl-substituted cyclopentadienyl groups, and R³ to R⁶ independently of one another denote hydrogen, C₁-C₄-alkyl, C₁-C₄-alkoxy, fluoromethyl, fluorine, aryl, phenoxy, nitrile or dialkylamino.

2. Process according to claim 1, **characterised in that** R¹ is a methoxycarbonyl group or methoxymethyl group.

3. Process according to claim 1 or 2, **characterised in that** the reaction is carried out in a non-polar or polar organic solvent.

4. Process according to any of claims 1 to 3, **characterised in that** palladium is used in the form of bis(triphenylphosphine)palladium(II) chloride or palladium(II) acetate.

5. Process according to any of claims 1 to 4, **characterised in that** the carbon monoxide pressure is 1 to 200 bar.

6. Process according to any of claims 1 to 5, **characterised in that** the reaction temperature is 100°C to 250°C.

## Revendications

1. Procédé de préparation d'esters d'acides pyridinecarboxyliques de formule générale 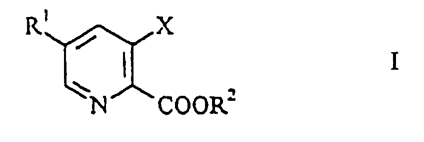 dans laquelle R¹ représente un atome d'hydrogène, un groupe alkyle en C₁-C₆, un groupe (alcoxy en C₁-C₄)carbonyle ou un groupe (alcoxy en C₁-C₄)méthyle, R² représente un groupe alkyle en C₁-C₄ et X représente le chlore ou le brome, **caractérisé en ce que** l'on fait réagir une 2,3-dihalogénopyridine de formule générale 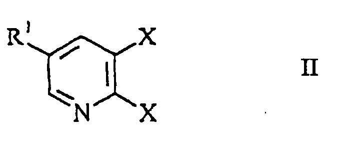 dans laquelle R¹ et X ont la signification indiquée ci-dessus, avec du monoxyde de carbone et un alcanol en C₁-C₄ en présence d'une base faible choisie dans le groupe constitué par des acétates, des hydrogénocarbonates et des hydrogénophosphates de métaux alcalins et de métaux alcalino-terreux, et d'un complexe de palladium avec une bisdiphénylphosphine de formule générale 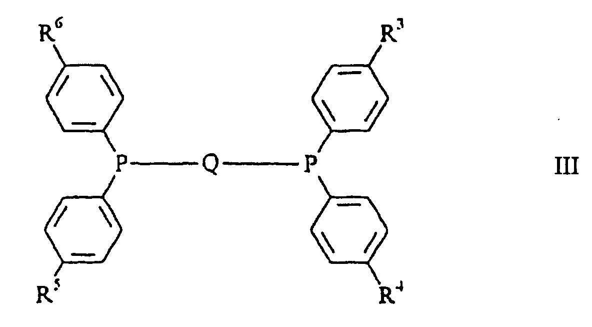 dans laquelle Q représente un groupe alcanediyle en C₃-C₆ ou un groupe 1,1'-ferrocènediyle dont les groupes cyclopentadiényle sont éventuellement substitués par alkyle en C₁-C₄ ou aryle, et les R³ à R⁶ représentent, indépendamment les uns des autres, un atome d'hydrogène ou un groupe alkyle en C₁-C₄, alcoxy en C₁-C₄, fluorométhyle, fluoro, aryle, phénoxy, nitrile ou dialkylamino.

2. Procédé selon la revendication 1, **caractérisé en ce que** R¹ est un groupe méthoxycarbonyle ou un groupe méthoxyméthyle.

3. Procédé selon la revendication 1 ou 2, **caractérisé en ce que** la réaction s'effectue dans un solvant organique non polaire ou polaire.

4. Procédé selon l'une quelconque des revendications 1 à 3, **caractérisé en ce que** l'on utilise le palladium sous forme de chlorure de bis(triphénylphosphine)-palladium-II ou d'acétate de palladium-II.

5. Procédé selon l'une quelconque des revendications 1 à 4, **caractérisé en ce que** la pression de monoxyde de carbone est de 1 à 200 bars.

6. Procédé selon l'une quelconque des revendications 1 à 5, **caractérisé en ce que** la température de la réaction est de 100 à 250°C.
